# EUROPEAN PATENT APPLICATION

(11) **EP 2 682 074 A1**
(43) Date of publication of application: **08.01.2014**
(21) Application number: 12751752.2
(22) Date of filing: 17.02.2012
(51) Int. Cl.: A61F 2/82, A61F 2/06, A61L 31/00

(54) **STENT DEVICE**

(30) Priority: 28.02.2011 JP 2011041275
(71) Applicant: Kyoto Medical Planning Co., Ltd., Kyoto-shi, Kyoto 607-8035 (JP)
(72) Inventor: IGAKI, Keiji, Kyoto-shi, Kyoto 607-8035 (JP); YAMADA, Hirokazu, Kyoto-shi, Kyoto 607-8035 (JP)
(74) Representative: Müller - Hoffmann & Partner
(86) International application number: PCT/JP2012/001066
(87) International publication number: WO 2012/117683

(57) **Abstract**

An object is to surely hold a stent formed of a biodegradable polymer and shape memorized to an expanded size having a diameter larger than an inner diameter of a vessel on a balloon in contracted state. A stent apparatus comprises a stent formed of a biodegradable polymer material using polylactic acid having a shape memory property as a tubular shape and shape memorized to a size capable of scaffolding a vessel from the inside when implanted in the vessel; and a stent holding member formed of a biodegradable polymer material for covering the outer surface of the stent to hold the stent in a contracted state that is smaller in diameter than the shape memorized size, wherein a stent detecting member is attached to the stent and a part of the stent holding member is fixed to the stent detecting member to prevent dislocation between the stent and the stent holding member.

## Description

### Technical field

This invention relates to a stent apparatus comprising a stent holding member for holding a stent, which is to be expanded in diameter when implanted in a vessel such as a blood vessel to scaffold the vessel from the inside, in a contracted state.

### Background Art

Heretofore, when stenosis occurs in a blood vessel, such as a coronary artery, stenting is performed in which the stenosed portion is expanded by using a medical balloon catheter and a cylindrical stent is implanted into this expanded portion to scaffold the vessel from the inside so as to ensure the blood flow.

As for this type of stent, the inventor of the present invention has proposed stents formed of a biodegradable polymer in WO92/15342(PTL 1), WO00/13737(PTL 2) and WO2009/157164(PTL 3). These stents are shape-memorized to an expanded size to scaffold a blood vessel from the inside, implanted in the blood vessel, and then heated by body temperature to expand from the contracted state to the expanded size for scaffolding the blood vessel from the inside.

This type of vascular stent is implanted to a blood vessel by using a catheter inserted into the blood vessel. At this time, the stent is mounted on a catheter and inserted into the blood vessel in a state contracted to a size having an outer diameter sufficiently smaller than the shape memorized size in order for smooth insertion without disengagement from the balloon.

It should be noted that, when the stent formed of the biodegradable polymer material is heated by body temperature and expanded to the shape memorized outer diameter, the diameter is gradually expanded over a period of time rather than immediately expanded due to the nature of the material. This is because the biodegradable polymer material has a property as a viscoelastic body and viscous resistance is produced when it expands to the shape memorized size. Since the expansion of the stent needs a certain length of time, implantation to a desired site might be failed due to dislocation affected by, for example, a blood flow during the process of expansion.

For this reason, the stent formed by using a biodegradable polymer material is delivered to an implantation position within the blood vessel, that is a lesion site, and then immediately expanded in diameter so that it is expanded to a size to scaffold the inner wall of the blood vessel by utilizing inflation force of a balloon capable of being rapidly expanded with injection of an expansion media.

The stent formed by using a biodegradable polymer material to be expanded by the above described balloon inflation force is mounted, in a contracted state, onto the balloon which has been mounted onto a tip of a catheter in a folded state, and is delivered to the implantation site together with this balloon. When delivered to the intended implantation site in the blood vessel, the stent is implanted at the lesion site by supplying the expansion media into the balloon and rapidly expanding the stent to a size to scaffold the blood vessel from the inside. The once expanded stent formed by using a biodegradable polymer material keeps the expansion to the shape memorized size by its self-expansion force even after the balloon is deflated by removal of the expansion medium, thereby scaffolding the implanted site from the inside to allow fluid path for blood in the blood vessel.

As described above, the stent formed by using a biodegradable polymer material having shape memory property and being shape-memorized to the expanded size to scaffold the blood vessel from the inside in an expanded state is heated by body temperature as it is inserted into the blood vessel, producing self-expansion force to recover the shape from contracted state to expanded state. By the effect of this shape recovery function, dislocation or disengagement might occur between the balloon and the stent mounted onto the balloon due to the friction force generated when the stent contacts with an inner wall of the blood vessel as it is inserted into the blood vessel. The stent disengaged from the balloon cannot be expanded in diameter with the balloon inflation force, making correct implantation to the intended site impossible. The stent not disengaged from the balloon but just dislocated relative to the balloon may be subjected to the balloon inflation force unequally along its entire length, resulting in its unequal expansion along its entire length. The stent expanded unequally along its length cannot scaffold the vessel wall in the vessel as intended.

In order to solve the above described problem, the inventors of the present invention has proposed a stent delivery apparatus wherein a balloon catheter, having a balloon on which the stent is mounted, is inserted into a protective sheath (WO2004/103450:PTL 4). Furthermore, in this stent delivery apparatus, a holding member holds the one end of the stent to prevent stent dislocation relative to the balloon, when the stent is extruded from the protective sheath to be expanded in diameter.

### Citation List

### Patent Literature

PTL1: WO92/15342
PTL2: WO00/13737
PTL3: WO2009/157164
PTL4: WO2004/103450

### Summary of Invention

### Technical Problem

In the above mentioned stent delivery apparatus, using the protective sheath for accommodating the balloon catheter mounting the stent on the balloon results in complex structure which is difficult to produce.

Furthermore, in the stent delivery apparatus using the protective sheath, operation for implanting a stent is cumbersome because, after the stent mounted on the balloon is inserted into a position around the implantation site, the protective sheath must be moved relative to the catheter so that the balloon protrudes from a tip of the protective sheath.

A technical object of the present invention is to provide a stent apparatus in which a stent can be surely mounted and held on a balloon of balloon catheters without using a protective sheath and the like, wherein the stent is formed of a biodegradable polymer material, shape-memorized to an expanded size larger than an inner diameter of a vessel when inserted into a vessel and heated by body temperature to recover the shape.

Another technical object of the present invention is to provide a stent apparatus capable of surely holding a stent mounted in a contracted state on a balloon of a balloon catheter, surely delivering the stent to an intended implantation site, and correctly implanting the stent at the implantation site by expanding the stent in diameter.

Another technical object of the present invention is to provide a stent apparatus in which a stent is capable of disappearing in a living body together with a member for keeping the stent in a contacted state, thereby eliminating the need to take them out from the living body.

Another technical object of the present invention is to provide a stent apparatus capable of holding a stent without inhibiting stent expansion conducted by balloon inflation while using a member for keeping the stent in a contracted state. Solution of Problem

To achieve the above-mentioned technical objects, the present invention provides a stent apparatus comprising: a stent formed of a biodegradable polymer material using polylactic acid (PLA) having a shape memory property as a tubular shape and shape memorized to a size capable of scaffolding a vessel from the inside when implanted in the vessel; and a stent holding member formed of a biodegradable polymer material for covering the outer surface of the stent to hold the stent in a contracted state that is smaller in diameter than the shape memorized size, wherein a stent detecting member is attached to the stent. A part of the stent holding member is fixed to the stent detecting member to prevent dislocation between the stent and the stent holding member.

The stent holding member is preferably formed of a biodegradable polymer material having a shape memory property and shape memorized to a contracted size having an inner diameter for holding the stent in the contracted state.

In the present invention, the stent is preferably formed of poly-L-lactide (PLLA) and the stent holding member is preferably formed of a copolymer of poly-L-lactide (PLLA) and poly-ε-caprolactone(PCL), shape memorized to a contracted size having an inner diameter to hold the stent in the contracted state, having an elasticity in the shape memorized state and having a property in which the elastic force thereof decreases as the diameter thereof is expanded from the shape memorized state.

In one embodiment, the stent holding member comprises a portion fixed to the stent detecting member by joining the portion to the stent detecting member with an adhesive.

The stent holding member may comprise a portion fixed to the stent detecting member by melting the portion and welding the melted portion to the stent detecting member.

Furthermore, the stent holding member may comprise an insertion hole opened therein and fixed to the stent detecting member by inserting the stent detecting member into the insertion hole.

In the case that the stent held by the stent holding member comprises an element in which linear parts and bend parts alternate in sequence and is expanded or contracted by altering opening angles of the bend parts, the stent detecting member for fixing the stent holding member is preferably attached to any one or more of the liner parts having a constant shape during expansion or contraction in diameter.

In the case that the stent held by the stent holding member is formed by combining a plurality of tubular body forming elements formed by bending a continuous strand such that linear parts and bend parts alternate in sequence and is expanded or contracted by altering opening angles of the bend parts, the stent detecting member for fixing the stent holding member is preferably attached to any one or more of the liner parts having a constant shape during expansion or contraction in diameter.

More preferably, the stent holding member comprises portions fixed to stent detecting members respectively attached to both ends of the stent formed as a tubular shape.

### Advantageous Effects of Invention

In the stent apparatus according to the present invention, a stent holding member for holding a stent in contracted state is fixed to a stent detecting member attached to the stent such that the stent apparatus can be kept in the unified state and transported to an intended implantation site without dislocation even if a force such as a friction force is applied to the stent holding member during the transportation through a vessel.

By shape memorizing the stent holding member to a contracted size having an inner diameter for holding the stent in a contracted state, the stent formed of a biodegradable polymer, the self-expansion force of which will gradually expand the diameter to recover the shape of the stent from the contacted state to the shape memorized size, will be further surely held in the contracted state, thereby eliminating the need for using a protective sheath and thus achieving a simplified apparatus structure.

The stent holding member for holding the stent has an elasticity in the state shape memorized to the size for holding the contracted state and has a property in which the elasticity decreases as it is expanded in diameter from the shape memorized size, so that the once expanded stent holding member will not recover the shape memorized state. The stent holding member therefore does not inhibit self-expansion force of the stent formed of a biodegradable polymer expanded from the contracted state to the shape memorized size, so that the expanding and supporting function of the stent against a vessel is guaranteed.

Joining the stent holding member to a stent detecting member with an adhesive surely unifies the stent holding member and the stent.

Furthermore, by melting a portion of the stent holding member and weld the melted portion to a stent detecting member, a stent apparatus with a high level of safety can be constituted without any effect of adhesive on a living body.

Moreover, opening an insertion hole in the stent holding member and inserting the stent detecting member into this insertion hole will facilitate the fixing therebetween.

When using a stent comprising an element in which linear parts and bend parts alternate in sequence and is expanded or contracted by altering opening angles of the bend parts, attaching the stent detecting member for fixing the stent holding member to any one or more of the liner parts having a constant shape during expansion or contraction in diameter will eliminate a possibility to produce a force which might restrict the deformation of the bend parts for altering opening angles during the expansion or contraction, so that stable expansion of the stent is guaranteed.

Similarly, when using a stent formed by combining a plurality of tubular body forming elements formed by bending a continuous strand such that linear parts and bend parts alternate in sequence and is expanded or contracted by altering opening angles of the bend parts, attaching the stent detecting member for fixing the stent holding member to any one or more of the liner parts having a constant shape during expansion or contraction in diameter will eliminate a possibility to produce a force which might restrict the deformation of the bend parts for altering opening angles during the expansion or contraction, so that stable expansion of the stent is guaranteed.

By fixing the stent and the stent holding member at a plurality of fixing portions, they can be surely transported to an intended implantation site while keeping the unified state therebetween.

Furthermore, by fixing the stent holding member at the both ends of the stent formed as a tubular shape, the stent and the stent holding member are unified in a further stable state.

### Brief Description of Drawings

(Fig.1) Fig.1 is a exploded perspective view of a stent apparatus according to the present invention.
(Fig.2) Fig.2 is a perspective view of a stent expanded to a size for implantation in a blood vessel.
(Fig.3) Fig.3 is a partial perspective view of a part of a stent to which a stent detecting member is attached.
(Fig.4) Fig.4 is a perspective view of a stent apparatus according to the present invention.
(Fig.5) Fig.5 is a cross sectional view of a stent apparatus according to the present invention.
(Fig.6) Fig.6 is a characteristic diagram showing elasticities and shape memory properties of the stent holding member and the stent.
(Fig.7A) Fig.7A is a partial cross sectional view showing a state in which a stent holding member is fixed to a stent detecting member.
(Fig.7B) Fig.7B is a partial plan view showing a state in which a stent holding member is fixed to a stent detecting member.
(Fig.8A) Fig.8A is a partial cross sectional view showing another example of a state in which a stent holding member is fixed to a stent detecting member.
(Fig.8B) Fig.8B is a partial plan view showing another example of a state in which a stent holding member is fixed to a stent detecting member.
(Fig.9A) Fig.9A is a partial cross sectional view showing another example of a state in which a stent holding member is fixed to a stent detecting member.
(Fig.9B) Fig.9B is a partial plan view showing another example of a state in which a stent holding member is fixed to a stent detecting member.
(Fig.10) Fig.10 is a cross sectional view showing a state in which a stent apparatus is mounted on a balloon of a balloon catheter.
(Fig.11) Fig.11 is a cross sectional view showing a state in which a stent is expanded together with the stent holding member by inflation of a balloon.
(Fig.12) Fig.12 is a cross sectional view showing a state in which a stent is implanted in a blood vessel.
(Fig.13) Fig.13 is a perspective view of another embodiment of a stent holding member.

### Description of Embodiments

Referring to attached drawings, embodiments of a stent apparatus according to the present invention are described hereinafter.

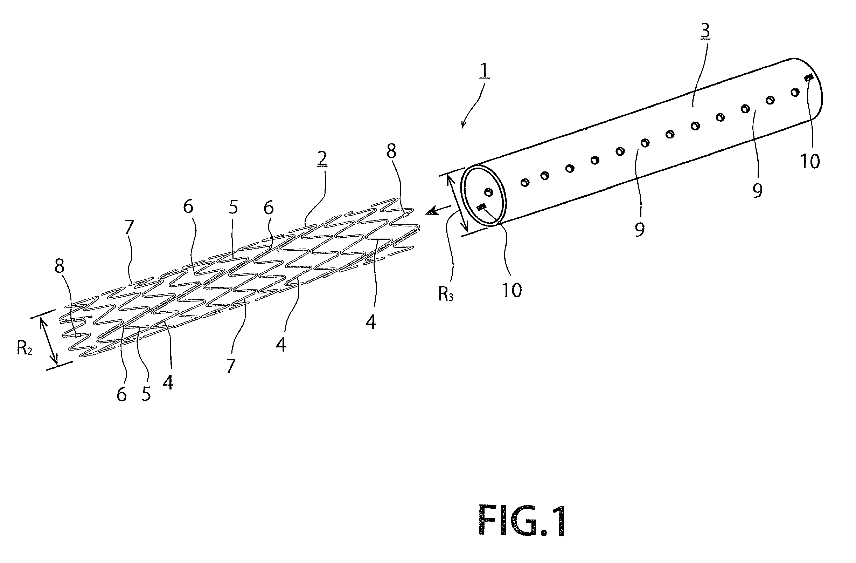

As shown in figure 1, the stent apparatus 1 according to the present invention includes a stent 2 formed of a biodegradable polymer material as a tubular shape, shape memorized to a size capable of, when implanted in a vessel of a living body such as an artery, scaffolding the blood vessel from the inside and a stent holding member 3 covering the stent 2 from the outside to hold the stent 2 in a contracted state smaller than this shape memorized size.

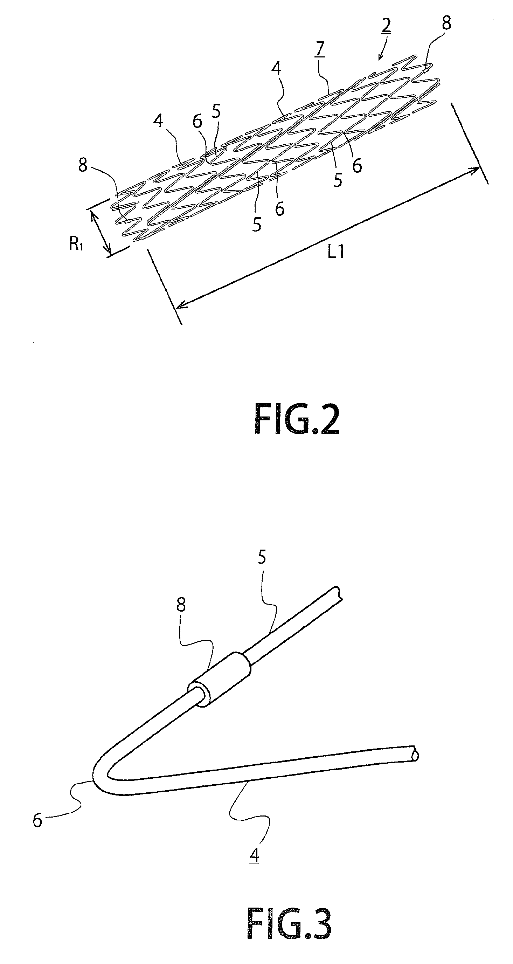

As shown in figure 2, the stent 2 used in this embodiment is constituted as a cylindrical body by combining tubular body forming elements 7 formed by bending a continuous strand 4 such that linear parts 5 and bend parts 6 alternate in sequence, to form a single channel from one side to the other side. The dimensions of this stent 2 are appropriately selected in accordance with a living body vessel such as a blood vessel in which the stent 2 is implanted. For example, the stent 2 constituted to be implanted in a blood vessel such as an artery is tubularly shaped wherein the outer diameter R1 is formed to be 2 to 5 mm and the length L1 is formed to be 10 to 40 mm as for the dimensions when implanted in the blood vessel. More generally, the stent 2 is formed to have an outer diameter to scaffold the blood vessel in which the stent 2 is implanted from the inside.

Furthermore, the stent 2 is formed of a biodegradable polymer so as not to exert bad influence on a living body when implanted into a living body such as a human body. As for this biodegradable polymer, a aliphatic polyester having a cross-linked structure realizing the shape memory property, more particularly, any one of polylactic acid (polylactide:PLA), polyglycolic acid (PGA), polyglactin (copolymer of polyglycolic acid and polylactic acid), polydioxanone, polyglyconate (copolymer of trimethylene carbonate and glycolide), and copolymer of polyglycolic acid or polylactic acid and ε-caprolactone can be used. Two or more of these materials can be compounded and used for this biodegradable polymer. Particularly, in view of safety to living bodies, it is desirable to use poly-L-lactide (PLLA) for this biodegradable polymer.

In the stent 2 formed by bending the continuous strand 4 in zigzag design such that linear parts 5 and bend parts 6 alternate in sequence and combining the tubular body forming elements 7, increasing the opening angles of the bend parts 6 of the strand 4 will result in expanded state having a larger diameter and decreasing the opening angles of the bend parts 6 will result in a contracted state having a smaller diameter.

As shown in figure 2, the stent 2 formed by combining plurality of the strands 4 bent in zigzag design is shape memorized to the expanded state in which the bend parts 6 of the strand 4 are opened to make the bend angles larger. The size to which the stent 2 is shape memorized is a size enough to scaffold a blood vessel from the inside when implanted in the blood vessel. The stent 2 is shape memorized by attaching the stent 2 onto a mold that keeps the expanded size of the stent 2, and then heat setting the mold. This heat setting is conducted with a temperature higher than the glass transition temperature of the biodegradable polymer constituting the stent 2.

The stent 2 shape memorized to the size to scaffold the blood vessel when implanted in the blood vessel is contracted to a size capable of being inserted into the blood vessel, as shown in figure 1. This contraction is conducted by applying a pressure to the outer peripheral of the tubularly formed stent 2. For example, this contraction is conducted by contract the expanded stent 2 and insert it into a tubular shaped mold having an inner diameter corresponding to the contracted size.

It should be noted that the stent 2 implanted in a living body is invisible by human eye. Stents made of metals can be located by using X-ray radiation devices. In contrast, the stent 2 is made of a biodegradable polymer such as PLLA having a high X-ray transmittance, making it difficult to detect an insertion position or implantation position within a blood vessel by using X-ray radiation. A stent detecting member 8 capable of being detected by X-ray radiation is therefore attached to the stent 2. This stent detecting member 8 is formed of a metal having a low X-ray transmittance and a stiffness higher than that of the biodegradable polymer. In this embodiment, the stent detecting member 8 is formed of gold having an excellent biocompatibility.

The stent detecting member 8 is made of a tubularly shaped metal component, through which a portion of the strand 4 is inserted as shown in figure 3.

In the stent 2 formed by combining plurality of the strands 4 bent in zigzag design, the stent detecting member 8 is preferably attached onto a linear part 5 having a constant shape during expansion or contraction in diameter.

This is also the same in the stent having an element wherein linear parts 5 and bend parts 6 alternate in sequence and constituted such that the diameter thereof is expanded or contracted by altering the opening angles of the bend parts.

By attaching stent detecting members 8 having a stiffness higher than that of the biodegradable polymer and thus unable to be easily deformed at linear parts 5 of the stent 2, stable expansion of the stent 2 is achieved since no force is applied which would restrict the deformation of the bend parts 6 for altering the opening angles when the stent 2 is expanded or contracted in diameter.

In this embodiment, two stent detecting members 8 are attached to both ends of the stent 2 respectively. The stent detecting members 8 attached to the both ends of the stent 2 facilitate recognition of implantation region of the stent 2 within a blood vessel.

It should be noted that, if only one stent detecting member 8 is provided, it is preferably attached at a center portion in longitudinal direction of the stent 2. By attaching the single stent detecting member 8 at the center portion in longitudinal direction of the stent 2, implantation region of the stent 2 can be identified by using preliminary knowledge of the length of the stent 2 implanted in the blood vessel.

In the stent 2 formed of a biodegradable polymer that is shape memorized to a predetermined size, even if the bend parts 6 are deformed by a pressure applied thereto such that the diameter of the stent 2 is contracted, self-expansion force will open the bend parts 6 to recover them from the contracted state to the shape memorized size when a temperature equal to or around the temperature having applied during the shape memory process. That is, when the stent 2 contracted from the shape memorized size is inserted into a living body and heated by body temperature, self-expansion force works to gradually expand the diameter from the contracted state to the shape memorized state. During transportation within a vessel, if this self-expansion force works to expand the diameter, the expansion of the stent 2 might trigger dislocation against the balloon, or disengagement from the balloon due to the friction force generated when the stent 2 contacts with an inner wall of the blood vessel.

In order to prevent these dislocation or disengagement, stent holding member 3 is used for holding the stent 2 in the contracted state. This stent holding member 3 is formed of a biodegradable polymer as a cylindrical shape having an inner diameter R3 smaller than the outer diameter R2 of the contracted stent 2 so as to hold the stent 2 in the contracted state. This stent holding member 3 is formed as the cylindrical shape by molding with a molding machine such as an injection molding machine.

The stent holding member 3 desirably holds the stent 2 on the balloon with a certain crimp force so as to prevent dislocation of the contracted stent 2 on the balloon of the balloon catheter. For this reason, stent holding member 3 is formed of a biodegradable polymer having a certain elasticity. Moreover, the stent holding member 3 is preferably formed of the same type of a biodegradable polymer as the stent 2. This is because excellent affinity will assure tighter contact between the stent 2 and the stent holding member 3, so that the stent 2 shape memorized to expanded state can be surely held in the contracted state. The stent covering member 3 in this embodiment therefore is formed of a copolymer of poly-L-lactide (PLLA) and poly-ε-caprolactone(PCL), which is a kind of aliphatic polyester. The copolymer of the PLLA and PCL used herein has a cross-linked structure and a shape memory property. In addition, the stent holding member 3 of the present embodiment is shape memorized to a contracted size having an inner diameter R3 to hold the stent 2 in the contracted state.

The stent holding member 3 is shape memorized by applying a heat setting to the stent holding member 3 molded by using an injection molding machine, for example. This heat setting is conducted by mounting the stent holding member 3 having an inner diameter R3 smaller than the outer diameter R2 of the contracted stent 2 onto a mold having an outer diameter smaller than the outer diameter R2 of the contracted stent 2 and applying a heat at a temperature higher than the glass transition temperature of the biodegradable polymer constituting the stent holding member 3.

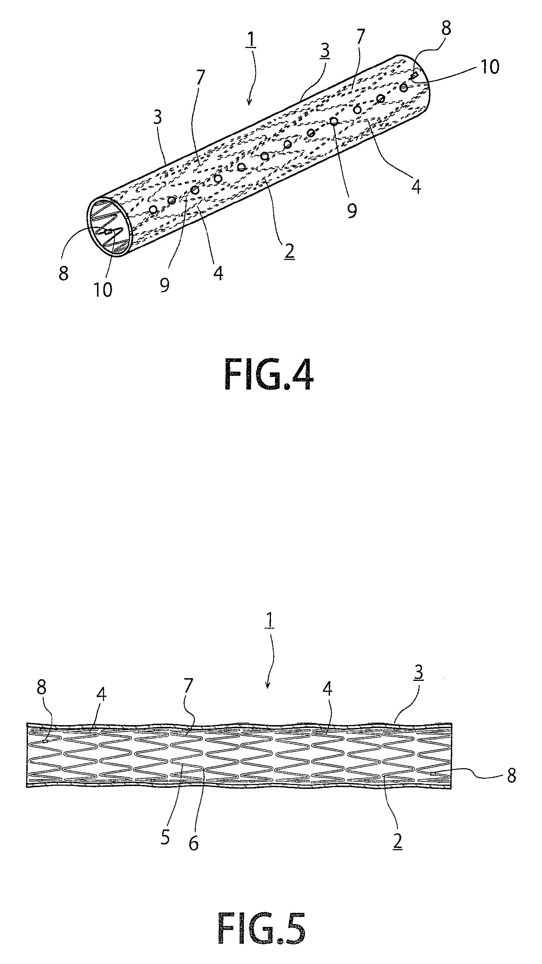

As shown in figure 4, the stent 2 is contracted in diameter to a size suitable for mounting onto a balloon and for transportation through a blood vessel, and then the outer surface of the stent 2 is covered with the stent holding member 3, which holds the stent 2 in a contracted state. In this embodiment, the stent holding member 3 is formed of a copolymer of PLLA and PCL, which is a biodegradable polymer material having a certain elastic force, thereby tightly crimping the outer surface of the stent 2 and elastically holding this stent 2 in the contracted state as shown in figure 5.

As shown in figure 1, a plurality of through holes 9 are parallelly opened along the longitudinal direction of the stent holding member 3 covering the stent 2 having a reticulate structure formed by bending the strand 4 to form the tubular body forming elements 7 and then combining the tubular body forming elements 7. These through holes 9 are provided in order that blood can contact with an inner wall of a blood vessel when this stent apparatus 1 is implanted at the lesion site of the blood vessel. The through holes 9 therefore may be of any shape including circular and rectangular shapes as long as it can transmit blood. The through holes 9 are desirably provided uniformly over the entire surface of the stent holding member 3.

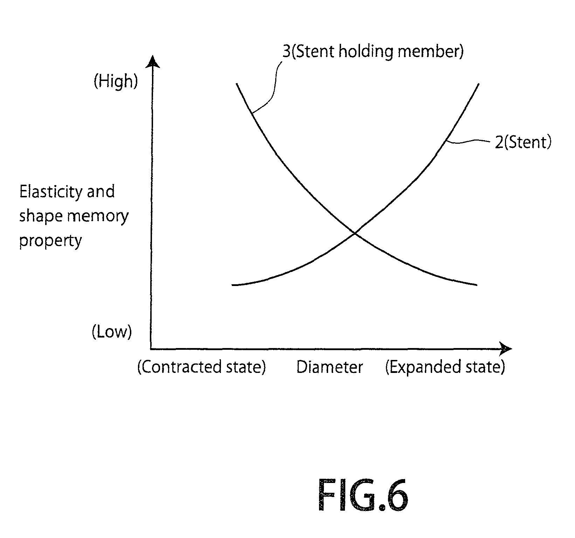

As stated above, the stent holding member 3 formed of a copolymer of PLLA and PCL and shape memorized to contracted size has a property in which elasticity and shape memory property decreases as it is expanded in diameter from the shape memorized size as shown in figure 6. On the other hand, the stent 2 formed of a biodegradable polymer material and shape memorized to expanded state has a property in which elasticity and shape memory property decrease as it is contracted in diameter from the shape memorized size as shown in figure 6. As will be apparent from the characteristic diagram shown in figure 6, the stent apparatus 1 according to the present invention can hold the stent 2 in a contracted state since the elasticity and shape memory property of the stent holding member 3 are larger than those of the stent 2 so as to keep the shape memorized size of the stent holding member 3.

The stent holding member 3 keeps the shape memorized contracted state when the stent 1 according to the present invention is mounted onto a balloon of a catheter and inserted into a blood vessel of a living body. At this time, since the elasticity and shape memory property of the stent holding member 3 is larger than those of the stent 2, the stent holding member 3 holds the stent 2 in a contracted state. Since the stent holding member keeps the contracted state, the stent apparatus 1 according to the present invention can keep the stent 2 in a contracted state even when it is inserted into a blood vessel in a living body and heated by body temperature, thereby preventing disengagement or dislocation from the balloon during transportation of the stent 2 mounted on the balloon of the catheter through the blood vessel.

Furthermore, when the stent holding member 3 is expanded from the shape memorized state by a physical force such as an expansion force of a balloon provided on a balloon catheter, the stent holding member 3 loses elasticity and shape memory property and is plastically deformed without elastic recovery as shown in figure 6. The stent holding member 3 therefore is plastically deformed without elastic recovery when the stent 2 mounted on a balloon is expanded by a certain amount in diameter by utilizing an expansion force of the balloon. When the stent holding member 3 is expanded to the extent capable of plastic deformation, expansion force of the stent 2 caused by heating is larger than contraction force of the stent holding member 3. The stent 2 heated by body temperature expands to the shape memorized size by self-expansion force thereof larger than contraction force of the stent holding member 3.

In the stent apparatus 1 according to the present invention, the stent holding member 3 shape memorized to a contracted size having an inner diameter for holding the stent 2 in a contracted state can hold the stent 2, which is shape memorized to an expanded size, in a contracted state. The expansion of the stent holding member 3 from the shape memorized state by a certain amount in diameter enables the expansion of the stent 2 shape memorized to the expanded size. The stent 2 therefore can be transported to an intended implantation site without disengagement or dislocation from the balloon during transportation and surely expanded at this implantation site to scaffold the blood vessel.

Moreover, in this invention, the stent holding member 3 is fixed to the stent detecting member 8 attached to the stent 2. This fixing is conducted by inserting the stent detecting member 8 into an insertion hole 10 opened in the stent holding member 3 and filling the insertion hole 10 with an adhesive 11 to join the stent holding member 3 to the stent detecting member 8.

This insertion hole 10 is provided at a position corresponding to the stent detecting member 8 when the stent 2 is covered. As for the adhesive 11, a solution in which a material constituting the stent holding member 3 is dissolved in a solvent can be used. This embodiment uses a solution in which a copolymer of poly-L-lactide (PLLA) and poly-ε-caprolactone(PCL), which is the material constituting the stent holding member 3, is dissolved in dioxane solution.

It should be noted that, in the case that plurality of stent detecting members 8 are attached to the stent 2, the insertion hole 10 are provided at a portion corresponding to the stent detecting members 8 attached at both ends of the stent 2. These stent holding member 3 are fixed at the both ends of the stent 2, respectively.

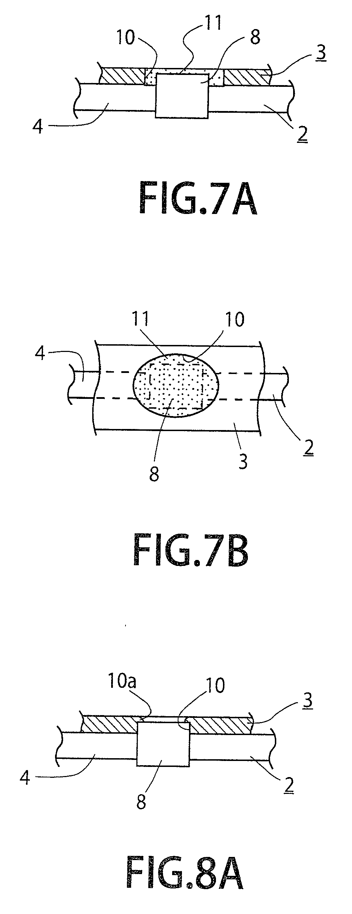

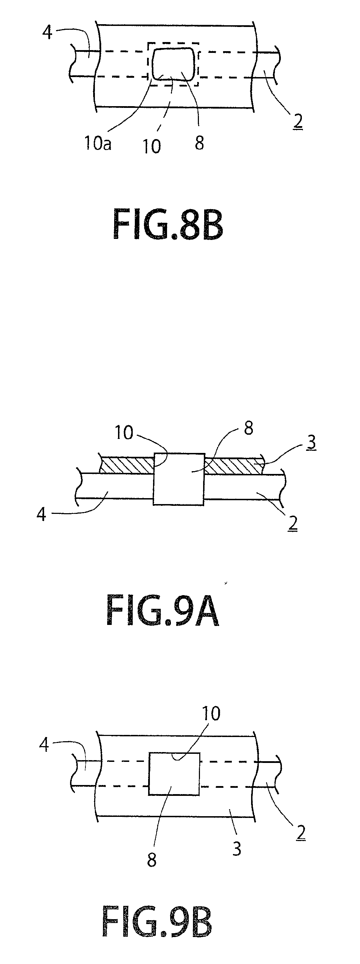

The fixing of the stent holding member 3 to the stent detecting member 8 may also be conducted by inserting the stent detecting member 8 into an insertion hole 10 opened in the stent holding member 3 and melting the periphery of the insertion hole 10 to weld a melted portion 10a of the stent holding member 3 to the stent detecting member 8, as shown in figures 8A and 8B.

Moreover, the stent holding member 3 may be fixed to the stent detecting member 8 by forming the insertion hole 10 to a size nearly equal to or slightly smaller than the stent detecting member 8 so as to realize tight fitting therebetween and inserting the stent detecting member 8 into this insertion hole 10 as shown in figures 9A and 9B.

As stated above, by fixing the stent holding member 3 to the stent detecting member 8 attached to the stent 2, even if a friction force is applied to the stent holding member 3 by contacting with an inner wall of the blood vessel, relative dislocation between the stent holding member 3 and the stent 2 is prevented such that the stent holding member 3 and the stent 2 can be surely unified and mounted on a balloon of a balloon catheter.

The stent holding member 3 can further surely hold the stent 2 in a contracted state on a balloon by shape memorizing the stent holding member 3 to a contracted size having an inner diameter for holding the stent 2 in a contracted state, such that the stent 2 can be surely transported to an intended implantation site without disengagement or dislocation from the balloon during transportation and correctly expanded at this implantation site to scaffold the blood vessel.

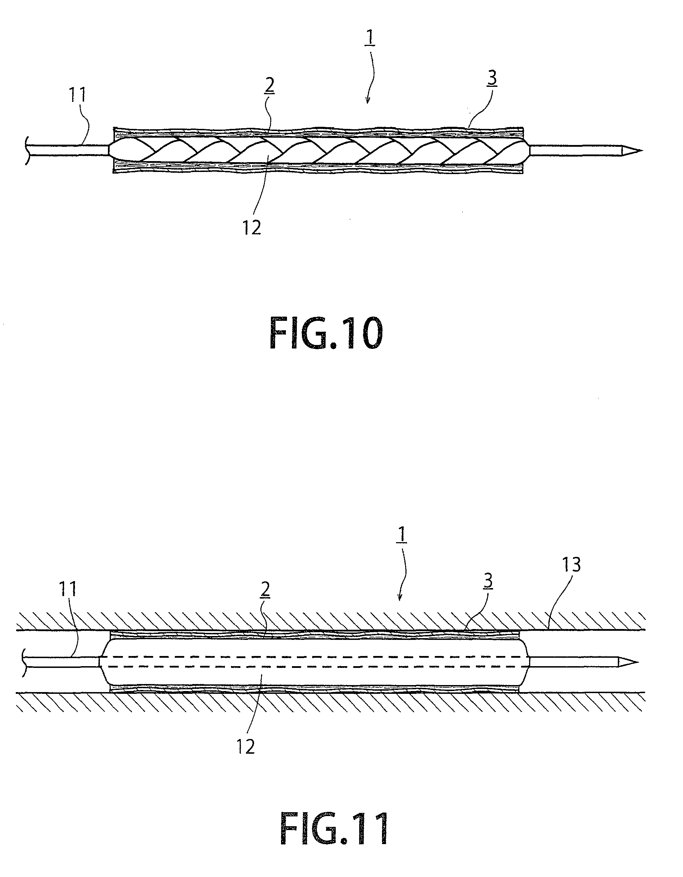

As shown in figure 10, the stent apparatus 1 constituted as stated above is mounted on a balloon 12 of a balloon catheter 11 together with the stent holding member 3. At this time, the stent apparatus 1 is tightly held on the balloon 12 with the elastic force of the stent holding member 3 in a contracted state.

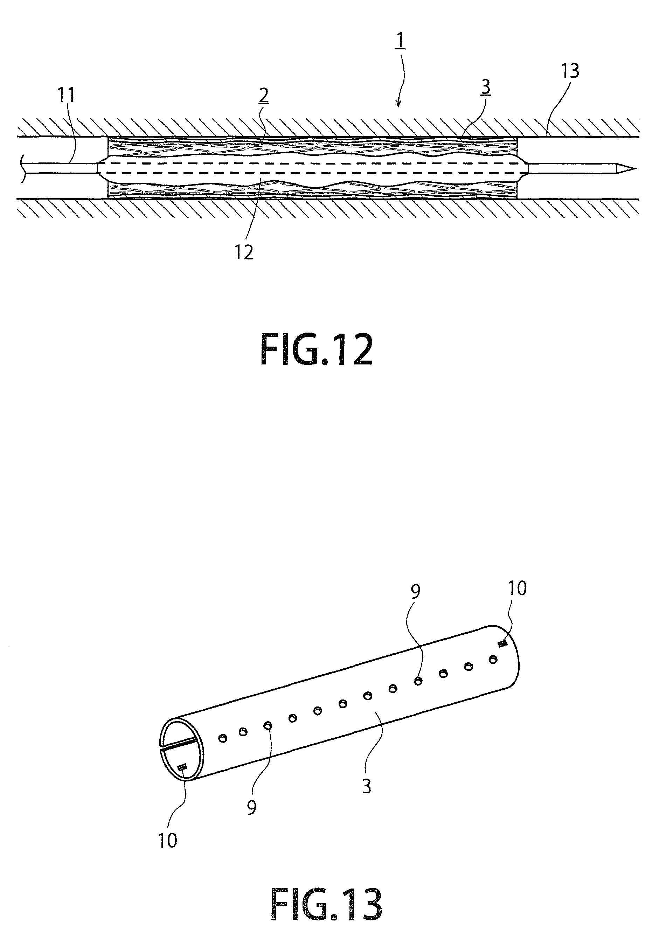

Then the stent apparatus 1 mounted on the balloon 12 is transported together with the balloon catheter 11 through a curved or serpentine blood vessel to a lesion site, which is the implantation site of the stent 2. The stent apparatus 1 transported to the lesion site is expanded by expanding the balloon 12, as shown in figure 11. When the stent 2 is expanded to a size equal to or around the shape memorized size by the expansion of the balloon 12, the stent holding member 3 loses elasticity and shape memory property thereof to the extent capable of plastic deformation. When the stent holding member 3 is expanded to the extent that it loses elasticity and shape memory property, the recovery force of the stent 2 to the shape memorized state is larger than the elastic force of the stent holding member such that the stent 2 is expanded to the shape memorized expanded size. Then the stent 2 expands and scaffolds an inner wall of a blood vessel 13 by self-expansion force thereof. Subsequently, the expansion medium supplied to the balloon 12 is drawn to deflate the balloon 12, as shown in figure 12, and removing the balloon catheter 11 from the blood vessel 13 completes the implantation process of the stent 2 in the blood vessel.

It should be noted that the stent holding member 3 may be of a tubular shaped sheet body comprising of a copolymer of PLLA and PCL. The shape of the stent holding member 3 is not limited to a tubular shape but may be of any shapes including a shape having a C-shaped cross section as shown in figure 13 as long as it can hold the outer surface of the stent 2 shape memorized to an expanded size larger than an inner diameter of a blood vessel in which the stent 2 will be implanted and keep this stent 2 in a contracted state. In this case, it is also desirable to provide a plurality of through holes 9. Similarly to the above, this stent holding member 3 is also provided with a insertion hole 10 such that inserting the stent detecting member 3 into this insertion hole 10 and joining them with an adhesive will fix the stent holding member 3 to the stent 2 and prevent dislocation therebetween.

Furthermore, the stent 2 used in this invention is not limited to the above stated stent formed by combining a plurality of tubular body forming elements 7 formed by bending a continuous strand 4 made of a biodegradable polymer. The present invention can be broadly applied to any stent including linear parts and bend parts alternating in sequence and capable of being expanded or contracted by altering the opening angles of the bend parts to obtain the advantages similar to those of described above.

As explained above, by employing the present invention, the stent shape memorized to an expanded size can be transported to an intended implantation site without disengagement or dislocation from the balloon during transportation and correctly expanded at this implantation site to scaffold the blood vessel.

### Reference Signs List

- 1: stent apparatus
- 2: stent
- 3: stent holding member
- 8: stent detecting member
- 9: through hole
- 10: insertion hole

## Claims

1. A stent apparatus comprising:
a stent formed of a biodegradable polymer material using polylactic acid (PLA) having a shape memory property as a tubular shape and shape memorized to a size capable of scaffolding a vessel from the inside when implanted in the vessel; and
a stent holding member formed of a biodegradable polymer material for covering the outer surface of the stent to hold the stent in a contracted state that is smaller in diameter than the shape memorized size, wherein
a stent detecting member is attached to the stent and a part of the stent holding member is fixed to the stent detecting member to prevent dislocation between the stent and the stent holding member.

2. The stent apparatus according to claim 1, wherein
the stent holding member is formed of a biodegradable polymer material having a shape memory property and shape memorized to a contracted size having an inner diameter for holding the stent in the contracted state.

3. The stent apparatus according to claim 1, wherein
the stent is formed of poly-L-lactide (PLLA) and
the stent holding member is formed of a copolymer of poly-L-lactide (PLLA) and poly-ε-caprolactone(PCL), shape memorized to a contracted size having an inner diameter to hold the stent in the contracted state, having an elasticity in the shape memorized state and having a property in which the elastic force thereof decreases as the diameter thereof is expanded from the shape memorized state.

4. The stent apparatus according to any one of claims 1 to 3, wherein
the stent holding member comprises a portion fixed to the stent detecting member by joining the portion to the stent detecting member with an adhesive.

5. The stent apparatus according to any one of claims 1 to 3, wherein
the stent holding member comprises a portion fixed to the stent detecting member by melting the portion and welding the melted portion to the stent detecting member.

6. The stent apparatus according to any one of claims 1 to 3, wherein
the stent holding member comprises a insertion hole opened therein and fixed to the stent detecting member by inserting the stent detecting member into the insertion hole.

7. The stent apparatus according to any one of claims 1 to 6, wherein
the stent comprises an element in which linear parts and bend parts alternate in sequence and is expanded or contracted by altering opening angles of the bend parts, and further wherein
the stent detecting member for fixing the stent holding member is attached to any one or more of the liner parts.

8. The stent apparatus according to any one of claims 1 to 6, wherein
the stent is formed by combining a plurality of tubular body forming elements formed by bending a continuous strand such that linear parts and bend parts alternate in sequence and is expanded or contracted by altering opening angles of the bend parts, and further wherein
the stent detecting member for fixing the stent holding member is attached to any one or more of the liner parts.

9. The stent apparatus according to any one of claims 1 to 8, wherein
the stent and the stent holding member are fixed at a plurality of fixing portions.

10. The stent apparatus according to any one of claims 1 to 8, wherein
the stent holding member comprises portions fixed to stent detecting members respectively attached to both ends of the stent formed as a tubular shape.
